# EUROPÄISCHE PATENTANMELDUNG

(11) **EP 2 774 482 A1**
(43) Veröffentlichungstag der Anmeldung: **10.09.2014**
(21) Anmeldenummer: 14157648.8
(22) Anmeldetag: 11.05.2010
(51) Int. Cl.: A01N 37/06, A01N 37/10, A01N 43/08, A01N 43/16, A01N 43/80, A61Q 19/00, A61K 8/49, A61Q 1/04, A61Q 1/14, A61Q 5/02, A61Q 5/06, A61Q 5/12, A61Q 15/00, A61Q 19/08, A61Q 19/10

(54) **Zusammensetzung enthaltend Sorbitanmonocaprylat und mindestens einen antimikrobiellen Wirkstoff ausgewählt aus bestimmten Isothiazolinonen**

(30) Priorität: 23.05.2009 DE 102009022444
(62) Teilanmeldung aus: 10722598.9
(71) Anmelder: Clariant Finance (BVI) Limited, Road Town, Tortola (VG)
(72) Erfinder: Pilz, Maurice, Frederic, 60329 Frankfurt am Main (DE); Scherl, Franz-Xaver, 84508 Burgkirchen (DE); Kluth, Giuseppina, 65779 Kelkheim (DE); Klug, Peter, Dr., 63672 Großostheim (DE); Gehm, Sonja, 65439 Flörsheim (DE)
(74) Vertreter: Jacobi, Markus Alexander

(57) **Zusammenfassung**

Die Erfindung betrifft flüssige Zusammensetzungen, die enthalten: 40 bis 99,9 Gew.% Sorbitanmonocaprylat und 0,1 bis 60 Gew.% von einem oder mehreren antimikrobiellen Substanzen, ausgewählt aus der Gruppe der Isothiazolinone, wobei die flüssigen Zusammensetzungen geeignet sind für die Herstellung von kosmetischen, dermatologischen oder pharmazeutischen Produkten.

## Beschreibung

Die vorliegende Erfindung betrifft flüssige Zusammensetzungen enthaltend Sorbitanmonocaprylat und antimikrobielle Wirkstoffe.

Kosmetische, dermatologische oder pharmazeutische Formulierungen und Produkte bieten im Allgemeinen auf Grund des hohen Wassergehalts, eines günstigen pH-Wertes zwischen pH 5-8 sowie einer begünstigenden Lagertemperatur von etwa 25 °C ein ideales Umfeld für das Wachstum von Mikroorganismen. Einige verwendete Inhaltsstoffe wie Proteine oder Pflanzenexktrakte können zusätzlich als Nahrungsquelle für Bakterien und Pilze dienen. Weiterhin können Mikroorganismen oder deren Sporen durch damit belastete Inhaltsstoffe in die Formulierung oder das Produkt gelangen.

Da eine kosmetische, dermatologische oder pharmazeutische Formulierung für den Anwender unbedenklich sein muss und durch eine mikrobielle Belastung eines solchen Produktes beispielsweise Hautreizungen bis hin zu ernsthaften Infektionen am Auge ausgelöst werden können, sollte eine kosmetische, dermatologische oder pharmazeutische Formulierung hinreichend gegen mikrobiellen Befall geschützt werden.

Dies wird im Allgemeinen durch den Zusatz von antimikrobiell wirkenden Substanzen, sogenannten Konservierungsmitteln, zu der Formulierung erreicht.

Das oder die eingesetzten antimikrobiellen Wirkstoffe sollten dabei so dosiert sein, dass das Wachstum von Mikroorganismen in der Formulierung oder dem Produkt nicht nur während der Lagerung verhindert wird, sondern auch eine Keimzahlvermehrung durch eine Neukontamination seitens des Anwenders durch sachgemäßen Gebrauch wirkungsvoll unterbunden wird.

Die antimikrobiellen Wirkstoffe sollen hierbei möglichst effizient Bakterien und Pilze abtöten, gleichzeitig aber mild und unbedenklich für den Menschen sein. Um letzteres zu gewährleisten, werden maximale Einsatzkonzentrationen und Anwendungsgebrauch wie -gebiete der antimikrobiellen Wirkstoffe, auf wissenschaftliche Untersuchungen gestützt, gesetzlich geregelt. In Europa gilt hierbei der ANNEX VI der Kosmetikverordnung.

Neue Erkenntnisse zum toxikologischen Potential eines antimikrobiellen Wirkstoffes führen zu einer Neubewertung seitens des Gesetzgebers. In den letzten Jahren hat dabei die Anzahl der Verwendungsverschärfungen von antimikrobiellen Wirkstoffen zugenommen.

Um den Verbraucher weiterhin gegen die durch mikrobiellen Befall auslösbaren Gefahren, aber auch vor den Nebenwirkungen von antimikrobiellen Wirkstoffen zu schützen, ist es von Vorteil, möglichst geringe Mengen an antimikrobiellen Wirkstoffen zu verwenden. Milde und unbedenkliche Wirkungsverstärker von antimikrobiellen Wirkstoffen bieten die Möglichkeit, die Einsatzkonzentration des antimikrobiellen Wirkstoffes noch weiter zu senken, ohne die Gefahr der Nebenwirkungen für den Menschen zu erhöhen.

Um die Gesamtmenge an antimikrobiellen Wirkstoffen in der kosmetischen, dermatologischen oder pharmazeutischen Formulierung gering zu halten, bestand die Aufgabe, eine dermatologisch und toxikologisch unbedenkliche Substanz zu finden, die die antimikrobielle Wirkung von antimikrobiellen Wirkstoffen unterstützt.

Überraschend wurde nun gefunden, dass das bereits in der Kosmetik als Tensid und emulgierendes Agens bekannte und verwendete Sorbitanmonocaprylat genau diese Bedingungen erfüllt.

Gegenstand der vorliegenden Erfindung sind daher flüssige Zusammensetzungen enthaltend
a) von 40 bis 99,9 Gew.-%, bevorzugt von 45 bis 99,5 Gew.-%, besonders bevorzugt von 50 bis 99 Gew.-% und insbesondere bevorzugt von 55 bis 98 Gew.-% Sorbitanmonocaprylat und
b) von 0,1 bis 60 Gew.-%, bevorzugt von 0,5 bis 55 Gew.-%, besonders bevorzugt von 1 bis 50 Gew.-% und insbesondere bevorzugt von 2 bis 45 Gew.-% an einem oder mehreren antimikrobiellen Wirkstoffen ausgewählt aus der Gruppe bestehend aus den Komponenten b1) bis b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b2) Formaldehyd-Donoren ausgewählt aus DMDM Hydantoin, Imidazolidinyl Urea, Diazolidinyl Urea und Sodium Hydroxymethylglycinate,
   b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
   b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben,
   b5) Pyridone und ihre Salze ausgewählt aus 1-Hydroxy-4-methyl-6-(2,4,4-trimethylpentyl)-2-pyridon und Piroctone Olamine.

Sorbitanmonocaprylat ist dermatologisch wie toxikologisch auch in sehr hohen Einsatzkonzentrationen unbedenklich und unterstützt die antimikrobielle Wirkung von antimikrobiellen Wirkstoffen.

Es wurde weiter gefunden, dass Sorbitanmonocaprylat nicht die Viskosität einer kosmetischen, dermatologischen oder pharmazeutischen Formulierung verringert, sondern sogar im Gegenteil leicht verdickende Eigenschaften besitzt. So können relativ hohe Mengen an Sorbitanmonocaprylat eingesetzt werden, ohne die Viskosität der kosmetischen, dermatologischen oder pharmazeutischen Formulierung zu erniedrigen oder eine Phasenseparation zu begünstigen.

Die für eine hinreichende Konservierung der kosmetischen, dermatologischen oder pharmazeutischen Formulierung benötigte Einsatzkonzentration von antimikrobiellen Wirkstoffen kann in der Kombination mit Sorbitanmonocaprylat signifikant verringert werden. Dadurch reicht oft die Verwendung eines antimikrobiellen Wirkstoffes zur Konservierung der kosmetischen, dermatologischen oder pharmazeutischen Formulierung aus.

Sorbitanmonocaprylat ist bei Raumtemperatur flüssig und mit anderen antimikrobiellen Wirkstoffen mischbar.

Vorteilhaft an den erfindungsgemäßen flüssigen und daher leicht handhabbaren Zusammensetzungen ist beispielsweise ihre gute Formulierbarkeit.

Vorzugsweise ist die eine oder sind die mehreren antimikrobiellen Wirkstoffe der Komponente b) ausgewählt aus der Gruppe bestehend aus den Komponenten b1), b3) und b5)
b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon und b5) Pyridone und ihre Salze ausgewählt aus 1-Hydroxy-4-methyl-6-(2,4,4-trimethylpentyl)-2-pyridon und Piroctone Olamine.

Insbesondere bevorzugt ist der eine oder sind die mehreren antimikrobiellen Wirkstoffe der Komponente b) ausgewählt aus der Gruppe bestehend aus den Komponenten b1), b3) und b5)
b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
b3) wässrigen Zusammensetzungen enthaltend von 20 bis 80 Gew.-%, bevorzugt von 30 bis 70 Gew.-% und besonders bevorzugt von 40 bis 60 Gew.-% Methylisothiazolinon,
b5) Piroctone Olamine.

Hierunter wiederum bevorzugt ist der eine oder sind die mehreren antimikrobiellen Wirkstoffe der Komponente b) ausgewählt aus der Gruppe bestehend aus den Komponenten b1) und b5)
b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
b5) Piroctone Olamine.

In einer außerordentlich bevorzugten Ausführungsform der Erfindung ist der eine oder sind die mehreren antimikrobiellen Wirkstoffe der Komponente b) ausgewählt aus der Gruppe der organischen Säuren und ihrer Salze bestehend aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen.

Ganz besonders bevorzugt ist der eine oder sind die mehreren antimikrobiellen Wirkstoffe der Komponente b) ausgewählt aus der Gruppe der organischen Säuren und ihrer Salze bestehend aus Benzoesäure, Sorbinsäure, 3-Acetyl-6-methyl-2[H]-pyran-2,4[3H]-dione (Dehydroacetic acid), p-Methoxybenzoesäure, Propionsäure, Milchsäure, Salicylsäure, Glykolsäure und ihren Salzen.

In einer weiteren außerordentlich bevorzugten Ausführungsform der Erfindung ist der antimikrobielle Wirkstoff der Komponente b) Piroctone Olamine.

In einer besonders bevorzugten Ausführungsform der Erfindung enthalten die erfindungsgemäßen flüssigen Zusammensetzungen ein oder mehrere weitere Substanzen ausgewählt aus
d) Wasser
e) antimikrobiellen Wirkstoffen und
f) Hydrotropen,
wobei die antimikrobiellen Wirkstoffe der Komponente e) und Hydrotrope der Komponente f) zu den antimikrobiellen Wirkstoffen der Komponente b) unterschiedlich sind.

Die antimikrobiellen Wirkstoffe der Komponente e) und die Hydrotrope der Komponente f) sind unterschiedlich zu Sorbitanmonocaprylat.

In einer besonders bevorzugten Ausführungsform der Erfindung enthalten die erfindungsgemäßen flüssigen Zusammensetzungen Wasser. In einer hierunter wiederum bevorzugten Ausführungsform der Erfindung ist das Wasser in einer Menge von 0,1 bis 35 Gew.-%, vorzugsweise von 0,1 bis 20 Gew.-% und besonders bevorzugt von 0,5 bis 10 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere weitere antimikrobielle Wirkstoffe, die zu den antimikrobiellen Wirkstoffen der Komponente b) unterschiedlich sind.

Diese weiteren antimikrobiellen Wirkstoffe, die zu den Verbindungen der Komponente b) unterschiedlich sind, sind vorzugsweise ausgewählt aus Alkoholen, wie beispielsweise Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin, Glycerin, lodopropynyl Butylcarbamat, 2-Bromo-2-Nitropropan-1,3-diol, Cetyltrimethylammoniumchlorid, Cetylpyridiniumchlorid, Benzethoniumchlorid, Diisobutylethoxyethyl-dimethylbenzylammoniumchlorid, Diisobutyl-phenoxyethoxy-ethyl-dimethylbenzyl-ammoniumchlorid, N-Alkyl-N,N-dimethyl-benzyl-ammoniumchlorid, -bromid, -saccharinat, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, 2-Hydroxybiphenyl, Chlorbutanulum, 5-Amino-1,3-bis-(2-ethylhexyl)-5-methyl-hexahydropyrimidin, 2,4-Dichlorbenzylalkohol, N-(4-Chlorphenyl-N'-(3,4-dichlorphenyl)harnstoff, 2,4,4'-Trichlor-2'-hydroxy-diphenylether, Poly(hexamethylendiguanid)-hydrochlorid, 1,2-Dibrom-2,4-dicyanobutan, 4,4-Dimethyl-1,3-oxazolidin, Chloroxylenol, Citratschwermetallsalzen, Silberchlorid, Piroctose, Pyrithionen und deren Schwermetallsalzen, insbesondere Zinkpyrithion, Zinkphenolsulfat, Farnesol, Bifonazole, Butoconazole, Cloconazole, Clotrimazole, Econazole, Enilconazole, Fenticonazole, Fluconazole, Isoconazole, Itraconazole, Ketoconazol, Miconazole, Naftifine, Oxiconazol, Sulconazole, Terbinafine, Terconazole und Tioconazole und Kombinationen dieser Wirksubstanzen.

In einer insbesondere bevorzugten Ausführungsform der Erfindung enthalten die erfindungsgemäßen flüssigen Zusammensetzungen ein oder mehrere weitere antimikrobielle Wirkstoffe, die zu den antimikrobiellen Wirkstoffen der Komponente b) unterschiedlich sind, und ausgewählt sind aus Alkoholen, vorzugsweise ausgewählt aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol, Ethylhexylglycerin und Glycerin und halogenierten Konservierungsstoffen, vorzugsweise ausgewählt aus lodopropynyl Butylcarbamat und 2-Bromo-2-Nitropropan-1,3-diol.

In einer außerordentlich bevorzugten Ausführungsform der Erfindung enthalten die erfindungsgemäßen flüssigen Zusammensetzungen einen oder mehrere weitere antimikrobielle Wirkstoffe, die zu den antimikrobiellen Wirkstoffen der Komponente b) unterschiedlich sind und ausgewählt sind aus Alkoholen, vorzugsweise ausgewählt sind aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol, Ethylhexylglycerin und Glycerin, besonders bevorzugt ausgewählt sind aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin und insbesondere bevorzugt ausgewählt sind aus der Gruppe der Alkohole bestehend aus Benzylalkohol, Phenoxyethanol, 1,2-Octandiol und Ethylhexylglycerin.

Sofern die erfindungsgemäßen flüssigen Zusammensetzungen ein oder mehrere weitere antimikrobielle Wirkstoffe, die zu den antimikrobiellen Wirkstoffen der Komponente b) unterschiedlich sind, enthalten, sind diese vorzugsweise in einer Menge von 0,5 bis 50 Gew.-%, besonders bevorzugt von 5 bis 45 Gew.-% und insbesondere bevorzugt von 10 bis 45 Gew.-% in den erfindungsgemäßen flüssigen Zusammensetzungen enthalten.

In dem Fall, dass die erfindungsgemäßen flüssigen Zusammensetzungen ein oder mehrere Salze von organischen Säuren enthalten, enthalten die erfindungsgemäßen flüssigen Zusammensetzungen vorzugsweise von 2 bis 35 Gew.-%, besonders bevorzugt von 5 bis 20 Gew.-% und insbesondere bevorzugt von 10 bis 15 Gew.-% Wasser.

In einer weiteren besonders bevorzugten Ausführungsform der Erfindung enthalten die erfindungsgemäßen flüssigen Zusammensetzungen ein oder mehrere Hydrotrope, die zu den Verbindungen der Komponente b) unterschiedlich sind. Diese Hydrotrope sind vorzugsweise ausgewählt aus Xylol-, Toluol- und Cumolsulfonat. Cumolsulfonat ist besonders bevorzugt.

Sofern die erfindungsgemäßen flüssigen Zusammensetzungen ein oder mehrere Hydrotrope, die zu den Verbindungen der Komponente b) unterschiedlich sind, enthalten, ist die Menge des einen oder der mehreren dieser Hydrotrope in den erfindungsgemäßen flüssigen Zusammensetzungen vorzugsweise im Bereich von 1 bis 15 Gew.-%, besonders bevorzugt von 4 bis 10 Gew.-% und insbesondere bevorzugt von 6 bis 8 Gew.-%.

In einer weiteren besonders bevorzugten Ausführungsform der Erfindung enthalten die erfindungsgemäßen flüssigen Zusammensetzungen ein oder mehrere weitere Additive.

Diese weiteren Additive sind vorzugsweise ausgewählt aus Antioxidantien und Solubilisatoren.

In einer weiteren besonders bevorzugten Ausführungsform der Erfindung enthalten die erfindungsgemäßen flüssigen Zusammensetzungen ein oder mehrere Antioxidantien.

Die Antioxidantien sind vorzugsweise ausgewählt aus Superoxid-Dismutase, Tocopherol (Vitamin E), Ascorbinsäure (Vitamin C), Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Äpfelsäure), Huminsäure, Phytinsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin-E-acetat), Vitamin A und Derivaten (Vitamin-A-palmitat), Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z.B. ZnO, ZnSO₄) Selen und dessen Derivaten (z. B. Selenmethionin), Stilbenen und deren Derivaten (z. B. Stilbenoxid, trans-Stilbenoxid) und Superoxid-Dismutase und erfindungsgemäß geeigneten Derivaten (Salze, Ester, Ether, Zucker, Nukleotide, Nukleoside, Peptide und Lipide) dieser genannten Stoffe.

Besonders bevorzugte Antioxidantien sind ausgewählt aus öllöslichen Antioxidantien.

Insbesondere bevorzugte Antioxidantien sind ausgewählt aus Tocopherylacetat, BHT (Butylhydroxytoluol) und EDTA.

Sofern die erfindungsgemäßen flüssigen Zusammensetzungen ein oder mehrere Antioxidantien enthalten, sind diese vorzugsweise in einer Menge von 0,001 bis 30 Gew.-%, besonders bevorzugt von 0,05 bis 20 Gew.-% und insbesondere bevorzugt von 0,1 bis 5 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere Solubilisatoren.

Bevorzugte Solubilisatoren sind Verbindungen ausgewählt aus der Gruppe bestehend aus Ethanol, Propanol, Isopropanol, n-Butanol, iso-Butanol, Butylenglykol, 1,2-Propylenglykol, Polyethylenglykolen mit einer relativen Molekülmasse von 300 bis 2000, insbesondere mit einer relativen Molekülmasse von 300 bis 600, Triacetin (Glycerintriacetat), 1-Methoxy-2-propanol und PEG-4-Laurat (Polyethylenglykol-4-Laurat).

Besonders bevorzugte Solubilisatoren sind ausgewählt aus Ethanol, Butylenglykol und 1,2-Propylenglykol und vorzugsweise aus Ethanol und 1,2-Propylenglykol. Ethanol ist insbesondere bevorzugt.

Sofern die erfindungsgemäßen flüssigen Zusammensetzungen ein oder mehrere Solubilisatoren enthalten, sind diese vorzugsweise in einer Menge von 1 bis 20 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen weniger als 5 Gew.-%, vorzugsweise weniger als 3 Gew.-% und besonders bevorzugt weniger als 1 Gew.-% Wasser. In einer insbesondere bevorzugten Ausführungsform der Erfindung enthalten die erfindungsgemäßen flüssigen Zusammensetzungen kein Wasser, d.h. sie sind wasserfrei.

Die in der vorliegenden Anmeldung für die erfindungsgemäßen flüssigen Zusammensetzungen angegebenen Mengen an Wasser stellen immer die Gesamtwassermenge in den erfindungsgemäßen flüssigen Zusammensetzungen dar. In dieser Gesamtwassermenge ist bereits die gegebenenfalls über Komponente b3) in die erfindungsgemäßen flüssigen Zusammensetzungen eingebrachte Menge an Wasser berücksichtigt.

In einer weiteren besonders bevorzugten Ausführungsform der Erfindung bestehen die erfindungsgemäßen flüssigen Zusammensetzungen aus
a) Sorbitanmonocaprylat und
b) einem oder mehreren der unter Komponente b) genannten antimikrobiellen Wirkstoffen.

In einer weiteren besonders bevorzugten Ausführungsform der Erfindung bestehen die erfindungsgemäßen flüssigen Zusammensetzungen aus
a) Sorbitanmonocaprylat,
b) einem oder mehreren der unter Komponente b) genannten antimikrobiellen Wirkstoffen und
d) Wasser.

In einer weiteren besonders bevorzugten Ausführungsform der Erfindung bestehen die erfindungsgemäßen flüssigen Zusammensetzungen aus
a) Sorbitanmonocaprylat,
b) einem oder mehreren der unter Komponente b) genannten antimikrobiellen Wirkstoffen und
e) einem oder mehreren weiteren antimikrobiellen Wirkstoffen, die zu den unter Komponente b) genannten antimikrobiellen Wirkstoffen unterschiedlich sind.

In einer weiteren besonders bevorzugten Ausführungsform der Erfindung bestehen die erfindungsgemäßen flüssigen Zusammensetzungen aus
a) Sorbitanmonocaprylat,
b) einem oder mehreren der unter Komponente b) genannten antimikrobiellen Wirkstoffen,
d) Wasser und
e) einem oder mehreren weiteren antimikrobiellen Wirkstoffen, die zu den unter Komponente b) genannten antimikrobiellen Wirkstoffen unterschiedlich sind.

Vorzugsweise besitzen die erfindungsgemäßen flüssigen Zusammensetzungen ein klares Aussehen.

In einer weiteren bevorzugten Ausführungsform der Erfindung sind die erfindungsgemäßen flüssigen Zusammensetzungen frei von Alkoholen R-OH, worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

Die erfindungsgemäßen flüssigen Zusammensetzungen sind in vorteilhafter Weise geeignet zum Konservieren kosmetischer, dermatologischer oder pharmazeutischer Produkte.

Weiterer Gegenstand der Erfindung ist daher die Verwendung einer erfindungsgemäßen flüssigen Zusammensetzung zum Konservieren von kosmetischen, dermatologischen oder pharmazeutischen Produkten, vorzugsweise von Cremes, Cremegelen, Lotionen, Shampoos, Duschbädern, Deodorantien, Antiperspirantien, Feuchttüchern (wet wipes), Sonnenschutzformulierungen oder dekorativen Kosmetikartikeln. In einer bevorzugten Ausführungsform der Erfindung werden kosmetische, dermatologische oder pharmazeutische Formulierungen konserviert.

Die erfindungsgemäßen flüssigen Zusammensetzungen sind des Weiteren in vorteilhafter Weise geeignet zur Herstellung von kosmetischen, dermatologischen oder pharmazeutischen Produkten, vorzugsweise von kosmetischen, dermatologischen oder pharmazeutischen Formulierungen.

Weiterer Gegenstand der vorliegenden Erfindung ist daher die Verwendung einer erfindungsgemäßen flüssigen Zusammensetzung zur Herstellung von kosmetischen, dermatologischen oder pharmazeutischen Produkten und vorzugsweise von kosmetischen, dermatologischen oder pharmazeutischen Formulierungen.

Unter dem Begriff "kosmetische, dermatologische oder pharmazeutische Produkte" werden im Rahmen der vorliegenden Erfindung beispielsweise entsprechende Formulierungen verstanden.

Bei den kosmetischen, dermatologischen oder pharmazeutischen Produkten kann es sich beispielsweise um wässrige, wässrig-alkoholische, wässrig-tensidische oder alkoholische Mittel oder um Mittel auf Ölbasis, inklusive Mittel auf Ölbasis in wasserfreier Form oder um Emulsionen, Suspensionen oder Dispersionen handeln und zwar in Form von Fluids, Schäumen, Sprays, Gelen, Mousse, Lotionen, Cremes, Pudern oder Feuchttüchern (Wet Wipes).

In einer bevorzugten Ausführungsform der Erfindung werden die erfindungsgemäßen flüssigen Zusammensetzungen zum Konservieren von Feuchttüchern verwendet. Hierbei kann es sich bei der auf das textile Gewebe aufgetragenen, zu konservierenden Formulierung um eine Emulsion, insbesondere eine O/W Emulsion, aber auch um eine tensidische Formulierung oder ein öliges Mittel handeln.

In einer weiteren bevorzugten Ausführungsform der Erfindung werden die erfindungsgemäßen flüssigen Zusammensetzungen zum Konservieren von Emulsionen verwendet.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d. h. beispielsweise durch Kalt-, Heiß-, Heiß/Kalt- oder PIT-Emulgierung erfolgen. Eine besonders bevorzugte Ausführungsform der Erfindung sind selbstschäumende, schaumförmige, nachschäumende oder schäumbare Emulsionen und Mikroemulsionen.

Weiterer Gegenstand der vorliegenden Erfindung sind kosmetische, dermatologische oder pharmazeutische Produkte, vorzugsweise kosmetische, dermatologische oder pharmazeutische Formulierungen, die unter Verwendung einer erfindungsgemäßen flüssigen Zusammensetzung hergestellt worden sind, die
a) Sorbitanmonocaprylat und
b) ein oder mehrere antimikrobielle Wirkstoffe ausgewählt aus der Gruppe bestehend aus den Komponenten b1) bis b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b2) Formaldehyd-Donoren ausgewählt aus DMDM Hydantoin, Imidazolidinyl Urea, Diazolidinyl Urea und Sodium Hydroxymethylglycinate,
   b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
   b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben, b5) Pyridone und ihre Salze ausgewählt aus 1-Hydroxy-4-methyl-6-(2,4,4-trimethylpentyl)-2-pyridon und Piroctone Olamine, enthält bzw. kosmetische, dermatologische oder pharmazeutische Produkte, vorzugsweise kosmetische, dermatologische oder pharmazeutische Formulierungen, die eine derartige erfindungsgemäße flüssige Zusammensetzung enthalten.

Weiterer Gegenstand der vorliegenden Erfindung sind kosmetische, dermatologische oder pharmazeutische Produkte, vorzugsweise kosmetische, dermatologische oder pharmazeutische Formulierungen, enthaltend
a) Sorbitanmonocaprylat und
b) ein oder mehrere antimikrobielle Wirkstoffe ausgewählt aus der Gruppe bestehend aus den Komponenten b1) bis b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b2) Formaldehyd-Donoren ausgewählt aus DMDM Hydantoin, Imidazolidinyl Urea, Diazolidinyl Urea und Sodium Hydroxymethylglycinate,
   b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
   b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben,
   b5) Pyridone und ihre Salze ausgewählt aus 1-Hydroxy-4-methyl-6-(2,4,4-trimethylpentyl)-2-pyridon und Piroctone Olamine.

Hierunter bevorzugt sind kosmetische, dermatologische oder pharmazeutische Produkte, vorzugsweise kosmetische, dermatologische oder pharmazeutische Formulierungen, enthaltend
a) Sorbitanmonocaprylat und
b) ein oder mehrere antimikrobielle Wirkstoffe ausgewählt aus der Gruppe bestehend aus den Komponenten b1) und b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b5) Piroctone Olamine.

In einer bevorzugten Ausführungsform der Erfindung enthalten die erfindungsgemäßen kosmetischen, dermatologischen oder pharmazeutischen Produkte, vorzugsweise die erfindungsgemäßen kosmetischen, dermatologischen oder pharmazeutischen Formulierungen,
a) Sorbitanmonocaprylat und
b1) ein oder mehrere organische Säuren und/oder ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen.

In einer weiteren bevorzugten Ausführungsform der Erfindung enthalten die erfindungsgemäßen kosmetischen, dermatologischen oder pharmazeutischen Produkte, vorzugsweise die erfindungsgemäßen kosmetischen, dermatologischen oder pharmazeutischen Formulierungen,
a) Sorbitanmonocaprylat und
b5) Piroctone Olamine.

In einer anderen bevorzugten Ausführungsform der Erfindung enthalten die erfindungsgemäßen kosmetischen, dermatologischen oder pharmazeutischen Produkte, vorzugsweise die erfindungsgemäßen kosmetischen, dermatologischen oder pharmazeutischen Formulierungen,
a) Sorbitanmonocaprylat,
b) ein oder mehrere antimikrobielle Wirkstoffe ausgewählt aus der Gruppe bestehend aus den Komponenten b1) bis b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b2) Formaldehyd-Donoren ausgewählt aus DMDM Hydantoin, Imidazolidinyl Urea, Diazolidinyl Urea und Sodium Hydroxymethylglycinate,
   b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
   b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben,
   b5) Pyridone und ihre Salze ausgewählt aus 1-Hydroxy-4-methyl-6-(2,4,4-trimethylpentyl)-2-pyridon und Piroctone Olamine, und
e) ein oder mehrere weitere antimikrobielle Wirkstoffe, die zu den unter Komponente b) genannten antimikrobiellen Wirkstoffen unterschiedlich sind.

Unter den genannten erfindungsgemäßen kosmetischen, dermatologischen oder pharmazeutischen Produkten, insbesondere den erfindungsgemäßen kosmetischen, dermatologischen oder pharmazeutischen Formulierungen, sind solche bevorzugt, in denen der eine oder die mehreren antimikrobiellen Wirkstoffe der Komponente e) ausgewählt sind aus Alkoholen, vorzugsweise ausgewählt aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol, Ethylhexylglycerin und Glycerin, und halogenierten Konservierungsstoffen, vorzugsweise ausgewählt aus lodopropynyl Butylcarbamat und 2-Bromo-2-Nitropropan-1,3-diol.

Besonders bevorzugt sind erfindungsgemäße kosmetische, dermatologische oder pharmazeutische Produkte, insbesondere erfindungsgemäße kosmetische, dermatologische oder pharmazeutische Formulierungen, enthaltend
a) Sorbitanmonocaprylat,
b) ein oder mehrere antimikrobielle Wirkstoffe ausgewählt aus der Gruppe bestehend aus den Komponenten b1) bis b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b2) Formaldehyd-Donoren ausgewählt aus DMDM Hydantoin, Imidazolidinyl Urea, Diazolidinyl Urea und Sodium Hydroxymethylglycinate,
   b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
   b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben,
   b5) Pyridone und ihre Salze ausgewählt aus 1-Hydroxy-4-methyl-6-(2,4,4-trimethylpentyl)-2-pyridon und Piroctone Olamine, und
e) einen oder mehrere Alkohole, vorzugsweise ausgewählt aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin.

Eine insbesondere bevorzugte Ausführungsform der Erfindung sind erfindungsgemäße kosmetische, dermatologische oder pharmazeutische Produkte, insbesondere erfindungsgemäße kosmetische, dermatologische oder pharmazeutische Formulierungen, enthaltend
a) Sorbitanmonocaprylat,
b) ein oder mehrere antimikrobielle Wirkstoffe ausgewählt aus der Gruppe bestehend aus den Komponenten b1) und b5)
   b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
   b5) Piroctone Olamine, und
e) einen oder mehrere Alkohole, vorzugsweise ausgewählt aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin.

Eine außerordentlich bevorzugte Ausführungsform der Erfindung sind erfindungsgemäße kosmetische, dermatologische oder pharmazeutische Produkte, insbesondere erfindungsgemäße kosmetische, dermatologische oder pharmazeutische Formulierungen, enthaltend
a) Sorbitanmonocaprylat,
b1) eine oder mehrere organische Säuren und/oder ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen und
e) einen oder mehrere Alkohole, vorzugsweise ausgewählt aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin.

Eine weitere außerordentlich bevorzugte Ausführungsform der Erfindung sind erfindungsgemäße kosmetische, dermatologische oder pharmazeutische Produkte, insbesondere erfindungsgemäße kosmetische, dermatologische oder pharmazeutische Formulierungen, enthaltend
a) Sorbitanmonocaprylat,
b5) Piroctone Olamine und
e) einen oder mehrere Alkohole, vorzugsweise ausgewählt aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin.

Bei den Salzen der einen oder mehreren der unter Komponente b1) genannten organischen Säuren handelt es sich vorzugsweise
bei Benzoesäure um Natriumbenzoat, Kaliumbenzoat oder Ammoniumbenzoat,
bei Sorbinsäure um Kaliumsorbat oder Ammoniumsorbat,
bei Dehydroacetic acid um Natrium Dehydroacetat, Kalium Dehydroacetat oder Ammonium Dehydroacetat,
bei p-Methoxybenzoesäure um Natrium p-Methoxybenzoat, Kalium p-Methoxybenzoat oder Ammonium p-Methoxybenzoat,
bei Ameisensäure um Natriumformiat, Kaliumformiat oder Ammoniumformiat, bei Essigsäure um Natriumacetat, Kaliumacetat oder Ammoniumacetat,
bei Propionsäure um Natriumpropionat, Kaliumpropionat, Ammoniumpropionat oder Calciumpropionat,
bei Milchsäure um Natriumlactat, Kaliumlactat, Ammoniumlactat oder Magnesiumlactat,
bei Undecensäure um Natriumundecylenat, Kaliumundecylenat, Ammoniumundecylenat, Magnesiumundecylenat oder Zinkundecylenat,
bei Salicylsäure um Natriumsalicylat, Kaliumsalicylat, Ammoniumsalicylat, Magnesiumsalicylat oder Zinksalicylat, und
bei Glykolsäure um Natriumglykolat, Kaliumglykolat, Ammoniumglykolat oder Magnesiumglykolat.

In einer insbesondere bevorzugten Ausführungsform der Erfindung sind die Substanzen der Komponenten a) und b), bezogen auf die fertigen erfindungsgemäßen kosmetischen, dermatologischen oder pharmazeutischen Produkte, vorzugsweise die fertigen erfindungsgemäßen kosmetischen, dermatologischen oder pharmazeutischen Formulierungen, gemeinsam zu 0,1 bis 4,0 Gew.-%, vorzugsweise gemeinsam zu 0,2 bis 3,0 Gew.-%, besonders bevorzugt gemeinsam zu 0,3 bis 2,5 Gew.-% und insbesondere bevorzugt gemeinsam zu 0,5 bis 2,0 Gew.-% in den Produkten bzw. Formulierungen enthalten.

In einer weiteren insbesondere bevorzugten Ausführungsform der Erfindung sind die Substanzen der Komponenten a), b) und e), bezogen auf die fertigen erfindungsgemäßen kosmetischen, dermatologischen oder pharmazeutischen Produkte, vorzugsweise die fertigen erfindungsgemäßen kosmetischen, dermatologischen oder pharmazeutischen Formulierungen, gemeinsam zu 0,1 bis 4,0 Gew.-%, vorzugsweise gemeinsam zu 0,2 bis 3,0 Gew.-%, besonders bevorzugt gemeinsam zu 0,3 bis 2,5 Gew.-% und insbesondere bevorzugt gemeinsam zu 0,5 bis 2,0 Gew.-% in den Produkten bzw. Formulierungen enthalten.

In einer weiteren bevorzugten Ausführungsform der Erfindung sind die erfindungsgemäßen kosmetischen, dermatologischen oder pharmazeutischen Produkte, vorzugsweise die erfindungsgemäßen kosmetischen, dermatologischen oder pharmazeutischen Formulierungen, frei von Alkoholen R-OH, worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

In einer weiteren bevorzugten Ausführungsform der Erfindung handelt es sich bei den erfindungsgemäßen kosmetischen, dermatologischen oder pharmazeutischen Produkten um Rinse-off Produkte, insbesondere um Shampoos, Haarspülungen, Haarkuren, Duschbäder, Duschgels oder Schaumbäder.

In einer weiteren bevorzugten Ausführungsform der Erfindung handelt es sich bei den erfindungsgemäßen kosmetischen, dermatologischen oder pharmazeutischen Produkten um Leave-on Produkte, insbesondere um Tagescremes, Nachtcremes, Pflegecremes, Nährcremes, Bodylotions, Salben oder Lippenpflegemittel. Weitere bevorzugte Leave-on Produkte sind dekorative Kosmetika, insbesondere Makeups, Eye-shadows, Lippenstifte oder Mascara.

In einer weiteren bevorzugten Ausführungsform der Erfindung handelt es sich bei den erfindungsgemäßen kosmetischen, dermatologischen oder pharmazeutischen Produkten um Sonnenschutzmittel. Diese enthalten einen oder mehrere UV-Filter auf organischer oder anorganischer Basis.

In einer weiteren bevorzugten Ausführungsform der Erfindung handelt es sich bei den erfindungsgemäßen kosmetischen, dermatologischen oder pharmazeutischen Produkten um Deodorantien und Antiperspirantien, insbesondere in Form von Sprays, Sticks, Gelen oder Lotionen.

In einer weiteren bevorzugten Ausführungsform der Erfindung handelt es sich bei den erfindungsgemäßen kosmetischen, dermatologischen oder pharmazeutischen Produkten um tensidfreie Mittel, insbesondere um tensidfreie feste Mittel oder um tensidfreie Emulsionen.

Die erfindungsgemäßen kosmetischen, dermatologischen oder pharmazeutischen Produkte, vorzugsweise die erfindungsgemäßen kosmetischen, dermatologischen oder pharmazeutischen Formulierungen, können als weitere Hilfs- und Zusatzstoffe Tenside, Emulgatoren, kationische Polymere, Verdicker, Filmbildner, antimikrobielle Wirkstoffe, Adstringentien, Antioxidantien, UV-Lichtschutzfilter, Pigmente/Mikropigmente, Gelierungsmittel, sowie weitere in der Kosmetik gebräuchliche Zusätze, wie z.B. Überfettungsmittel, feuchtigkeitsspendende Mittel, Silicone, Stabilisatoren, Konditioniermittel, Glycerin, Konservierungsmittel, Perlglanzmittel, Farbstoffe, Duft- und Parfümöle, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistika, Antiallergica, Corticosteroide, Sebostatika), Vitamine, Bisabolol^{®}, Allantoin^{®}, Phytantriol^{®}, Panthenol^{®}, AHA-Säuren (alpha-Hydroxysäuren), Pflanzenextrakte, beispielsweise Aloe Vera und Proteine enthalten.

In einer bevorzugten Ausführungsform der Erfindung handelt es sich bei den erfindungsgemäßen kosmetischen, dermatologischen oder pharmazeutischen Produkten um entsprechende Formulierungen.

Weiterer Gegenstand der vorliegenden Erfindung ist die Verwendung von Sorbitanmonocaprylat zur Verbesserung der antimikrobiellen Wirksamkeit eines oder mehrerer antimikrobieller Wirkstoffe ausgewählt aus der Gruppe bestehend aus den Komponenten b1) bis b5)
b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
b2) Formaldehyd-Donoren ausgewählt aus DMDM Hydantoin, Imidazolidinyl Urea, Diazolidinyl Urea und Sodium Hydroxymethylglycinate,
b3) Isothiazolinone ausgewählt aus wässrigen Zusammensetzungen enthaltend von 20 bis 80 Gew.-%, bevorzugt von 30 bis 70 Gew.-% und besonders bevorzugt von 40 bis 60 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon,
b4) Parabenester und ihre Salze ausgewählt aus Methylparaben, Natrium Methylparaben, Ethylparaben, Natrium Ethylparaben, Propylparaben, Natrium Propylparaben, Isobutylparaben, Natrium Isobutylparaben, Butylparaben und Natrium Butylparaben,
b5) Pyridone und ihre Salze ausgewählt aus 1-Hydroxy-4-methyl-6-(2,4,4-trimethylpentyl)-2-pyridon und Piroctone Olamine.

Bevorzugt ist die Verwendung von Sorbitanmonocaprylat zur Verbesserung der antimikrobiellen Wirksamkeit eines oder mehrerer antimikrobieller Wirkstoffe ausgewählt aus der Gruppe bestehend aus den Komponenten b1) und b5)
b1) organische Säuren und ihre Salze ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen,
b5) Piroctone Olamine.

Besonders bevorzugt ist die Verwendung von Sorbitanmonocaprylat zur Verbesserung der antimikrobiellen Wirksamkeit einer oder mehrerer organischer Säuren und/oder ihrer Salze ausgewählt aus der Gruppe bestehend aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und ihren Salzen.

Weiterhin besonders bevorzugt ist die Verwendung von Sorbitanmonocaprylat zur Verbesserung der antimikrobiellen Wirksamkeit von Piroctone Olamine.

In einer weiteren bevorzugten Ausführungsform der Erfindung sind unter den organischen Säuren und ihren Salzen der Komponente b1) Benzoesäure und ihre Salze bevorzugt.

Die Herstellung der erfindungsgemäßen flüssigen Zusammensetzungen kann beispielsweise durch Zusammengeben der einzelnen Komponenten, gegebenenfalls unter Erwärmen auf ca. 80 °C, erfolgen.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

### Beispiele:

### I) Erfindungsgemäße flüssige Zusammensetzungen

### Beispiele 1 -15

Zusammensetzungen bestehend aus
1) 90 % Sorbitanmonocaprylat, 10 % Benzoesäure
2) 50 % Sorbitanmonocaprylat, 15 % Dehydroacetic acid, 20 % Methylparaben, 15 % Ethanol
3) 40 % Sorbitanmonocaprylat, 20 % Methylparaben, 20 % Propylparaben, 20 % Ethanol
4) 60 % Sorbitanmonocaprylat, 15 % Kaliumsorbat, 20 % Glyoxalsäure, 5 % Wasser
5) 90 % Sorbitanmonocaprylat, 5 % Piroctone Olamine, 5 % Ethanol
6) 80 % Sorbitanmonocaprylat, 5 % Piroctone Olamine, 1 % 50%ige Lösung von Methylisothiazolinone in Wasser, 14 % Ethanol
7) 70 % Sorbitanmonocaprylat, 30 % DMDM Hydantoin
8) 55 % Sorbitanmonocaprylat, 15 % Methylparaben, 20 % DMDM Hydantoin, 10 % Ethanol
9) 55 % Sorbitanmonocaprylat, 30 % Milchsäure, 5 % Piroctone Olamine, 10 % Dehydroacetic acid
10) 50 % Sorbintanmonocaprylat, 15 % Methylparaben, 15 % Ethylparaben, 5 % Piroctone Olamine, 1 % 50 %ige Lösung von Methylisothiazolinone in Wasser, 14 % Ethanol
11) 75 % Sorbitanmonocaprylat, 10 % Sodium Hydroxymethylglycinat, 10 % Propylparaben, 5 % Propylenglykol
12) 40 % Sorbitanmonocaprylat, 35 % 1,2-Octandiol, 10 % Kaliumsorbat, 10 % Phenoxyethanol, 5 % Wasser
13) 50 % Sorbitanmonocaprylat, 40 % Benzylalkohol, 5 % Piroctone Olamine, 1 % 50 %ige Lösung von Methylisothiazolinone in Wasser, 4 % Ethanol
14) 50 % Sorbitanmonocaprylat, 30 % Benzylalkohol, 15 % Benzoesäure, 5 % Piroctone Olamine
15) 99 % Sorbitanmonocaprylat, 1 % 50 %ige Lösung von Methylisothiazolinone in Wasser

Die Herstellung der Zusammensetzungen der Beispiele 1 bis 15 erfolgte, indem die einzelnen Komponenten unter Rühren nacheinander am Fingerrührer bei Rührgeschwindigkeiten von 200-300 Umdrehungen/Minute vermengt wurden. Teils und besonders bei Zugabe von organischen Säuren wurde die Zusammensetzung auf etwa 50-80 °C erwärmt, um eine homogene Mischung zu erhalten.

### II) Untersuchung der Wirksamkeitsverstärkung durch Sorbitanmonocaprylat

Nachfolgende Beispiele zeigen Ergebnisse von Challenge Tests, die nach den Vorgaben der Ph.Eur. Kapitel 5.1.3 durchgeführt wurden. Hierbei werden als Stellvertreter für Mikroorganismen folgende Testkeime verwendet: *Pseudomonas aeruginosa* (Gram-negativ; im Folgenden als "P.a." abgekürzt), *Staphylococcus aureus* (Gram-positiv; im Folgenden als "St.a." abgekürzt), *Candida albicans* (Hefepilz; im Folgenden als "C.a." abgekürzt) und *Aspergillus brasitiensis* (Schimmelpilz; im Folgenden als "A.b." abgekürzt). Bei einem Challenge Test gilt eine kosmetische, dermatologische oder pharmazeutische Formulierung genau dann als ausreichend konserviert, wenn alle vier Testkeime zumindest mit einem B-Kriterium bestanden werden. Ein A-Kriterium bedeutet eine hervorragende Konservierung, ein B-Kriterium bedeutet eine ausreichende Konservierung und mit einem F-Kriterium gilt der Test insgesamt als nicht bestanden. Als Vergleich wird ein unkonserviertes Muster der jeweiligen Formulierung mit ausgetestet. Im Folgenden gelten die weiteren Abkürzungen SC = Sorbitanmonocaprylat, Octopirox^{®} = Piroctone Olamine

### Beispiel 16

Als Testsystem dient hier eine schwer zu konservierende Creme (Formulierung A).

### Formulierung A

| Zutaten (INCI) | Gew.-% |
|---|---|
| Water | ad 100 % |
| Vitis Vinifera Seed Oil *(Grape seed oil*) | 6,0 % |
| Caprylic/Capric Triglyceride | 6,0 % |
| Glycerin | 3,0 % |
| Cetearyl Alcohol | 3,0 % |
| Glyceryl Stearate | 3,0 % |
| Prunus Amygdalus Dulcis Oil *(Almond oil*) | 2,5 % |
| Cetearyl Glucoside | 2,0 % |
| Prunus Armeniaca Kernel Oil *(Apricot Kernel Oil*) | 1,5 % |
| Simmondsia Chinensis Oil *(Jojoba Oil*) | 1,3 % |
| Lecithin | 1,0 % |
| Guar Gum | 0,5 % |
| Xanthan Gum | 0,5 % |
| Sodium Citrate | 1,0 % |
| Tocopherol Acetate | 0,5 % |
| Allantoin | 0,3 % |
| Panthenol | 0,3 % |

### Beispiel 16a)

In einer Konzentration von 0,4 % reicht Benzoesäure als einzelner antimikrobieller Wirkstoff zur ausreichenden Konservierung von Formulierung A nicht aus. Die in der EU erlaubte maximale Einsatzkonzentration von Benzoesäure für leave-on Produkte beträgt 0,5 %. Durch Zusatz von Sorbitanmonocaprylat kann die Creme jedoch bei gleichbleibender Konzentration der Benzoesäure konserviert werden (s. Tabelle A).

**Tabelle A Konservierung der Formulierung A mit Benzoesäure und Mischungen aus Benzoesäure und Sorbitanmonocaprylat (SC)**

| Antimikrobielle Wirkstoffe | pH | Testsystem | Einsatzkonzentration | Ergebnis | | | |
|---|---|---|---|---|---|---|---|
| | | | | P.a. | St.a. | C.a. | A.b. |
| Benzoesäure | 5,5 | Creme | 0,4 % | A | F | B | B |
| Benzoesäure + SC | " | " | 0,4 % + 0,5 % | A | F | B | A |
| Benzoesäure + SC | " | " | 0,4 % + 1,0 % | A | B | B | A |
| Benzoesäure + SC | " | " | 0,4 % + 1,5 % | A | A | A | A |
| unkonserviert | " | " | --- | F | F | F | F |

| | | | | | | | |
|---|---|---|---|---|---|---|---|
| P.a. = Pseudomonas aeruginosa C.a. = Candida albicans St.a. = Staphylococcus aureus A.b. = Aspergillus brasiliensis | | | | | | | |

### Beispiel 16b)

Das gleiche Testsystem (Formulierung A) kann durch Piroctone Olamine (Octopirox^{®}) in einer Einsatzkonzentration von 0,05 % nicht ausreichend konserviert werden. Durch Zugabe von Sorbitanmonocaprylat kann die Creme ausreichend gegen das Wachstum von Bakterien geschützt werden (s. Tabelle B).

**Tabelle B Konservierung der Formulierung A mit Octopirox^{®} und Mischungen aus Octopirox^{®} und Sorbitanmonocaprylat (SC)**

| Antimikrobielle Wirkstoffe | pH | Testsystem | Einsatzkonzentration | Ergebnis | | | |
|---|---|---|---|---|---|---|---|
| | | | | P.a. | St.a. | C.a. | A.b. |
| Octopirox^{®} | 5,5 | Creme | 0,05 % | F | F | F | F |
| Octopirox^{®} + SC | " | " | 0,05 % + 0,5 % | A | F | F | F |
| Octopirox^{®} + SC | " | " | 0,05 % + 1,0 % | A | F | F | F |
| Octopirox^{®} + SC | " | " | 0,05 % + 1,5 % | A | B | F | F |
| unkonserviert | " | " | --- | F | F | F | F |

| | | | | | | | |
|---|---|---|---|---|---|---|---|
| P.a. = Pseudomonas aeruginosa C.a. = Candida albicans St.a. = Staphylococcus aureus A.b. = Aspergillus brasiliensis | | | | | | | |

### Beispiel 17

Als Testsystem dient hier ein schwer zu konservierendes Shampoo mit Milchprotein (Formulierung B).

| Formulierung B | |
|---|---|
| Zutaten (INCI) | Gew.-% |
| Sodium Laureth Sulfate | 13,70 % |
| Water | ad 100 % |
| Coco Betaine | 6,00 % |
| Sodium Chloride | 1,40 % |
| Hydrolyzed Milk Protein | 1,00 % |

Piroctone Olamine (Octopirox^{®}) kann in einer Einsatzkonzentration von 0,05 % die Formulierung B nicht ausreichend gegen das Wachstum von Schimmelpilzen schützen. Dies kann durch Zugabe von Sorbitanmonocaprylat erreicht werden. Hierbei werden auch bessere Ergebnisse bei dem Gram-positiven Bakterium erzielt (s. Tabelle C).

**Tabelle C Konservierung der Formulierung B mit Octopirox^{®} und Mischungen aus Octopirox^{®} und Sorbitanmonocaprylat (SC)**

| Antimikrobielle Wirkstoffe | pH | Test-system | Einsatz-konzentration | Ergebnis | | | |
|---|---|---|---|---|---|---|---|
| | | | | P.a. | St.a. | C.a. | A.b. |
| Octopirox^{®} | 5,5 | Shampoo | 0,05 % | A | B | A | F |
| Octopirox^{®} + SC | " | " | 0,05 % + 0,5 % | A | B | A | F |
| Octopirox^{®} + SC | " | " | 0,05 % + 1,0 % | A | B | A | F |
| Octopirox^{®} + SC | " | " | 0,05 % + 1,5 % | A | A | A | F |
| Octopirox^{®} + SC | " | " | 0,05 % + 2,0 % | A | A | A | B |
| unkonserviert | " | " | --- | F | F | A | F |

| | | | | | | | |
|---|---|---|---|---|---|---|---|
| P.a. = Pseudomonas aeruginosa C.a. = Candida albicans St.a. = Staphylococcus aureus A.b. = Aspergillus brasiliensis | | | | | | | |

Die Vergabe der A-, B- und F- Kriterien richtet sich nach der Höhe der Keimzahlreduktion innerhalb eines festgelegten Zeitintervalls, wobei nur die logarithmische Reduktion betrachtet wird. Eine Verringerung der Keimzahl konnte auch in Beispiel 17 bei der Zugabe von 0,5 % oder 1,0 % Sorbitanmonocaprylat zu 0,05 % Octopirox^{®} im Vergleich zur alleinigen Verwendung von 0,05 % Octopirox^{®} beobachtet werden, obwohl sich dies nicht in den in Tabelle C aufgeführten A-, B- und F-Kriterien wiederspiegelt.

### III) Kosmetische Formulierungen enthaltend erfindungsgemäße flüssige Zusammensetzungen

Von jeder der im Folgenden aufgeführten kosmetischen Formulierungen A - M wurden jeweils 15 verschiedene Formulierungen hergestellt. Und zwar wurde jede kosmetische Formulierung A - M jeweils unter Verwendung der einzelnen erfindungsgemäßen flüssigen Zusammensetzungen der Beispiele 1 bis 15 ("Blends 1-15") hergestellt.

### Beispiel A - Shampoo

| | | | |
|---|---|---|---|
| A | Genapol^{®} LRO Paste | Clariant | 13,70 % |
| | *Sodium Laureth Sulfate* | | |
| | Genagen^{®} KB | Clariant | 6,00 % |
| | *Coco Betaine* | | |
| | Water | | ad 100 % |
| | | | |
| B | Sodium Chloride | | 1,50 % |
| | | | |
| C | Erfindungsgemäßer Blend 1-15 | Clariant | 1,50 % |
| | | | |
| D | Citric Acid (10 % in water) | | 0,08 % |

### Herstellung:

- I: Mische die Komponenten von A
- II: Gebe B unter Rühren zu I
- III: Gebe C zu II
- IV: Passe den pH-Wert auf ungefähr 7 an

### Beispiel B - Gesichtsreiniger

| | | | |
|---|---|---|---|
| A | Genapol^{®} LRO liquid | Clariant | 11,10 % |
| | *Sodium Laureth Sulfate* | | |
| | Parfume | | q.s. |
| | | | |
| B | Water | | ad 100 % |
| | Genagen^{®} 3SB | Clariant | 23,30 % |
| | Coco *Betaine, Sodium Cocoyl Isethionate, Sodium* | | |
| | *Methyl Cocoyl Taurate* | | |
| | Dyestuff solution | | q.s. |
| | | | |
| C | Erfindungsgemäßer Blend 1-15 | Clariant | 1,20 % |
| | | | |
| D | Citric Acid | | q.s. |

### Herstellung:

- I: Mische die Komponenten A
- II: Gebe die Komponenten von B nach einander in I
- III: Gebe C zu II unter Rühren
- IV: Falls gewünscht, passe den pH-Wert mit C an

### Beispiel C - Aftershave Gel

| | | | |
|---|---|---|---|
| A | Emulsogen^{®} HCU | Clariant | 1,50 % |
| | *Undeceth-8 (and) PEG-40 Hydrogenated Castor Oil* | | |
| | | | |
| B | Tocopherolacetat | | 0,20 % |
| | Menthol | | 0,20 % |
| | | | |
| C | Ethanol | | 30,00 % |
| D | Water | | ad 100 % |
| | Allantoin | Clariant | 0,20 % |
| | *Allantoin* | | |
| | Polyglykol 400 | Clariant | 3,00 % |
| | *PEG-8* | | |
| | Polyglykol 35000 | Clariant | 1,00 % |
| | *PEG-800* | | |
| | Erfindungsgemäßer Blend 1-15 | Clariant | 2,00 % |
| | | | |
| E | Aristoflex^{®} AVC | Clariant | 1,00 % |

| | | | |
|---|---|---|---|
| *Ammonium Acryloyldimethyltaurate*/*VP Copolymer* | | | |

### Herstellung:

- I: Mische A und B und rühre für etwa 5 Minuten
- II: Gebe C zu I und rühre bis die Lösung klar ist
- III: Gebe die Komponenten von D nach einander zu II
- IV: Gebe E zu I und rühre bis eine homogene Formulierung erhalten wird

### Beispiel D - Anti-Ageing Gesichtscreme

| | | | |
|---|---|---|---|
| A | Genapol^{®} T 250 | Clariant | 1,50 % |
| | *Cetereth-25* | | |
| | Genapol^{®} DAT | Clariant | 2,00 % |
| | *PEG-150 Polyglyceryl-2 Tristearate and PEG-6 Caprylic*/*Capric Glyceride* | | |
| | | | |
| B | Water | | ad 100 % |
| | | | |
| C | Aristoflex^{®} AVC | Clariant | 2,00 % |
| | *Ammonium Acryloyldimethyltaurate*/*VP Copolymer* | | |
| D | Glycolic Acid 30 % * | | 6,00 % |
| | Erfindungsgemäßer Blend 1-15 | Clariant | 1,80 % |

| | | | |
|---|---|---|---|
| * mit NaOH auf pH 4 eingestellt (Gehalt basiert auf freier Glykolsäure) | | | |

### Herstellung:

I Löse A in B unter Rühren und leichtem Erwärmen
II Gebe C zu I und rühre bis das entstehende Gel frei von Klümpchen ist
III Gebe die Komponenten von D zu II und rühre bis die Formulierung homogen ist

### Beispiel E - Emulsion für Baby Wet Wipes

| | | | |
|---|---|---|---|
| A | Propylene Glycol | | 3,00 % |
| | Erfindungsgemäßer Blend 1-15 | Clariant | 2,00 % |
| | Emulsogen^{®} HCO 040 | Clariant | 1,00 % |
| | *PEG-40 Hydrogenated Castor Oil* | | |
| | Parfume | | 0,20 % |
| | | | |
| B | Hostaphat^{®} KL 340 D | Clariant | 1,50 % |
| | *Trilaureth-4 Phosphate* | | |
| | Velsan^{®} CCT | Clariant | 0,80 % |
| | *Caprylic*/*Capric Triglyceride* | | |
| | | | |
| C | Water | | ad 100 % |
| | Tetrasodium EDTA | | 0,10 % |
| | | | |
| D | Aristoflex® BLV | Clariant | 0,20 % |
| | *Ammonium Acryloyldimethyltaurate*/*Beheneth- 25 Methacrylate Crosspolymer* | | |
| | | | |
| E | Citric Acid | | q.s. |

### Herstellung:

- I: Löse die Komponenten von A
- II: Gebe die Komponenten von B nach einander unter Rühren zu I
- III: Mische die Komponenten von C
- IV: Gebe D zu II
- V: Gebe unter Rühren III zu IV
- VI: Passe den pH-Wert mit E auf etwa pH 6 an

### Beispiel F - O/W Body Lotion

| | | | |
|---|---|---|---|
| A | Velsan^{®} CCT | Clariant | 3,50 % |
| | *Caprylic*/*Capric Triglyceride* | | |
| | Myristyl Myristate | | 2,50 % |
| | Cetearyl Alcohol | | 2,00 % |
| | Glyceryl Stearate Citrate | | 1,00 % |
| | Octyldodecanol | | 1,00 % |
| | | | |
| B | Aristoflex^{®} AVC | Clariant | 0,60 % |
| | *Ammonium Acryloyldimethyltaurate*/*VP Copolymer* | | |
| | | | |
| C | Water | | ad 100 % |
| | Glycerin | | 7,50 % |
| | | | |
| D | Ethanol | | 3,00 % |
| | Dimethicone | | 3,00 % |
| | Tocopheryl Acetate | | 1,00 % |
| | Aloe Barbadensis | | 1,00 % |
| | Erfindungsgemäßer Blend 1-15 | Clariant | 2,00 % |
| | | | |
| E | Sodium Hydroxide | | q.s. |

### Herstellung:

- I: Schmelze die Komponenten von A bei etwa 70 °C
- II: Mische die Komponenten von C und erhitze die Mischung auf etwa 70 °C
- III: Gebe B zu I wenn I vollständig geschmolzen ist
- IV: Gebe II zu III
- V: Bei 35 °C gebe die Komponenten von D zu IV
- VI: Stelle den pH-Wert mit E auf etwa pH 6,0-6,5 ein

### Beispiel G - Antiperspirant

| | | | |
|---|---|---|---|
| A | Locron^{®} L | Clariant | 30,00 % |
| | *Aluminum Chlorohydrate* | | |
| | Water | | ad 100 % |
| | Polyglykol 400 | Clariant | 3,00 % |
| | *PEG-8* | | |
| | Ethanol | | 17,00 % |
| | Dyestuff solution | | q.s. |
| | Erfindungsgemäßer Blend 1-15 | Clariant | 1,00 % |
| | Fragrance | | 0,30 % |
| | | | |
| B | Tylose^{®} H 4000 G4 | | 2,50 % |
| | *Hydroxyethlycellulose* | | |

### Herstellung:

I Mische die Komponenten von A
II Gebe B unter ständigem Rühren zu I. Rühre so lange weiter, bis die Viskosität ihren Endpunkt erreicht hat und die Formulierung homogen ist.

### Beispiel H - Cremespülung

| | | | |
|---|---|---|---|
| A | Genamin^{®} DSAP | Clariant | 2,50 % |
| | *Distearyldimonium Chloride* | | |
| | Genamin^{®} CTAC | Clariant | 3,00 % |
| | *Cetrimonium Chloride* | | |
| | Hostacerin^{®} T- 3 | Clariant | 1,50 % |
| | *Ceteareth- 3* | | |
| | Cetyl Alcohol | | 3,00 % |
| | | | |
| B | Water | | ad 100 % |
| | Erfindungsgemäßer Blend 1-15 | Clariant | 1,00% |
| | | | |
| C | Fragrance | | 0,30 % |
| | Dyestuff solution | | q.s. |

### Herstellung:

- I: Schmelze A bei etwa 75 °C
- II: Erhitze B auf etwa 75 °C
- III: Gebe II unter Rühren zu I und rühre bis zum Abkühlen auf 30 °C
- IV: Bei etwa 30 °C gebe C unter Rühren zu II

### Beispiel I - Haarstyling Gel

| | | | |
|---|---|---|---|
| A | Sorbitol | | 5,00 % |
| | Genamin^{®} PQ 43 | Clariant | 0,30 % |
| | Polyquaternium - 43 | | |
| | | | |
| B | Water | | ad 100 % |
| | | | |
| C | Aristoflex^{®} HMB | Clariant | 2,00 % |
| | *Ammonium Acryloyldimethyltaurate*/ *Beheneth- 25 Methacrylate Crosspolymer* | | |
| | | | |
| D | Aminomethyl Propanol | | 0,30 % |
| | Aristoflex^{®} A 60 | Clariant | 5,00 % |
| | *VA*/*Crotonates Copolymer* | | |
| | Emulsogen^{®} HCO 040 | Clariant | 4,00 % |
| | *PEG-40 Hydrogenated Castor Oil* | | |
| | | | |
| E | Fragrance | | 0,20 % |
| | Erfindungsgemäßer Blend 1-15 | Clariant | 1,00 % |
| | Dyestuff solution | | q.s. |
| | Timiron Diamond Cluster MP- 149 | | q.s. |
| | *Mica (and) Titanium Dioxide (for EU: CI 77891)* | | |

### Herstellung:

- I: Mische die Komponenten von A
- II: Gebe B zu I
- III: Quelle C in II unter Rühren auf
- IV: Gebe die Komponenten von D eine nach der anderen zu
- V: Gebe die Komponenten von E eine nach der anderen zu IV

### Beispiel J - Make Up Entferner

| | | | |
|---|---|---|---|
| A | Velsan^{®} P8-3 | Clariant | 5,00 % |
| | *Isopropyl C12-15 Pareth-9 Carboxylate* | | |
| | | | |
| B | Hostapon^{®} KCG | Clariant | 2,30 % |
| | *Sodium Cocoyl Glutamate* | | |
| | Genagen^{®} CAB | Clariant | 3,00 % |
| | *Cocamidopropyl Betaine* | | |
| | Genapol^{®} LA 070 | Clariant | 2,00 % |
| | *Laureth-7* | | |
| | Water | | ad 100 % |
| | Allantoin | Clariant | 0,30 % |
| | *Allantoin* | | |
| | Aristoflex^{®} PEA | Clariant | 1,00 % |
| | *Polypropylene Terephthalate* | | |
| | 1,6 Hexanediol | | 2,00 % |
| | 1,2 Propanediol | | 2,00 % |
| | Polyglykol 400 | Clariant | 2,00 % |
| | *PEG-8* | | |
| | Panthenol | | 0,50 % |
| | Lutrol F 127 | | 3,00 % |
| | *Poloxamer 407* | | |
| | Erfindungsgemäßer Blend 1-15 | Clariant | 1,70 % |

### Herstellung:

I Rühre die Komponenten von B nach einander in A und rühre bis eine klare Lösung erhalten wird

### Beispiel K - Lippenglanz

| | | | |
|---|---|---|---|
| A | Versagel^{®} ME 1600 | | ad 100 % |
| | *Hydrogenated polyisobutene (and)* | | |
| | *Ethylene*/*Propylene*/ *Styrene Copolymer (and)* | | |
| | *Buylene*/*Ethylene*/*Styrene Copolymer* | | |
| | SilCare^{®} Silicone 31M50 | Clariant | 7,00 % |
| | *Caprylyl Trimethicone* | | |
| | SilCare^{®} Silicone 41 M65 | Clariant | 3,00 % |
| | *Stearyl Dimethicone* | | |
| | Jojoba Oil | | 2,60 % |
| | Velsan CCT | Clariant | 1,00 % |
| | *Capric*/*Caprylic Triglycerides* | | |
| | Isopropyl Myristate | | 7,40 % |
| | | | |
| B | Gemtone^{®} Tan Opal | | 1,00 to 5,00 % |
| | *Mica and Iron Oxide and TiO₂* | | |
| | Lake - Color | | q.s. |
| | | | |
| C | Erfindungsgemäßer Blend 1-15 | Clariant | 0,50 % |
| | | | |
| D | Parfum | | q.s. |

### Herstellung:

- I: Erhitze die Komponenten von A auf etwa 80-85 °C und rühre so lange, bis eine homogene Mischung erhalten wird. Lasse diese Mischung auf 70-75 °C abkühlen
- II: Gebe B und C nacheinander unter Rühren zu I und rühre, bis alle Bestandteile gelöst sind
- III: Lasse bis auf 45 °C abkühlen und gebe D zu II, dann fülle die Formulierung in die Gussformen

### Beispiel L - Shimmering Bronze Gel

| | | | |
|---|---|---|---|
| A | Water | | ad 100 % |
| | | | |
| B | Glycerin | | 5,00 % |
| | Polyglykol 35000 S | Clariant | 0,50 % |
| | *PEG-800* | | |
| | Allantoin | Clariant | 0,20 % |
| | *Allantoin* | | |
| C | Aristoflex^{®} AVC | Clariant | 0,60 % |
| | *Ammonium Acryloyldimethyltaurate*/*VP Copolymer* | | |
| | Biron MTU | | 3,00 % |
| | *Bismuth Oxychloride* | | |
| | Flamenco Ultra Silk | | 4,00 % |
| | *Titanium Oxide (and) Mica* | | |
| | Flamenco Sparcle Gold | | 7,00 % |
| | *Mica (and) Iron Oxide (and) Titanium Oxide* | | |
| | Cloisonné Satin Bronze | | 5,00 % |
| | *Iron Oxide (and) Mica* | | |
| | Gemtone Sunstone | | 2,00 % |
| | *Mica (and) Iron Oxide (and) Titanium Oxide* | | |
| | Desert Reflections Canyon Sunset | | 2,00 % |
| | *Mica (and) Iron Oxide (and) Titanium Oxide (and)* | | |
| | *Tin Oxide* | | |
| | SilCare^{®} Silicone WSI | Clariant | 1,00 % |
| | *proposed INCI: Glyceryl Carboxy Amodimethicone* | | |
| | | | |
| D | Fragrance | | q.s. |
| | Erfindungsgemäßer Blend 1-15 | Clariant | 1,80 % |

### Herstellung:

- I: Mische die Komponenten von B und löse sie unter Rühren in A
- II: Mische die Komponenten von C und gebe unter leichtem Rühren zu I
- III: Rühre mit höherer Umdrehungszahl (etwa 200 - 250 Umdrehungen/Minute) für etwa zwei Stunden oder bis ein homogenes Gel erhalten wird
- IV: Gebe D unter Rühren zu III

### Beispiel M - Sonnencreme

| | | | |
|---|---|---|---|
| A | SilCare^{®} Silicone WSI | Clariant | 2,00 % |
| | *proposed INCI: Glyceryl Carboxy Amodimethicone* | | |
| | SilCare^{®} Silicone 41 M65 | Clariant | 1,00 % |
| | *Stearyl Dimethicone* | | |
| | Dow Corning^{®}246 | | 11,00 % |
| | *Cyclopentasiloxane*/*Cyclohexasiloxane* | | |
| | Titandioxid UV Titan M 262 | | 10,00 % |
| | *Titanium Dioxide*/*Dimethicone* | | |
| | Solaveil CT-100 | | 10,00 % |
| | *C12-15 Alkyl Benzoate*/*Titanium* | | |
| | *Dioxide*/*Aluminium Stearate*/ *Polyhydroxystearic Acid*/*Alumina* | | |
| | *Z*-Cote HP1 | | 8,00 % |
| | *Zinc Oxide* | | |
| | Butylene Glycol | | 3,00 % |
| | Hostacerin^{®} DGI | Clariant | 3,00 % |
| | *Polyglyceryl-2 Sesquiisostearate* | | |
| | Tegosoft^{®} TN | | 2,00 % |
| | *C12-15 Alkyl Benzoate* | | |
| | Cetiol^{®} 868 | | 2,00 % |
| | *Ethylhexylstearate* | | |
| | | | |
| B | Water | | ad 100 % |
| | Glycerin | | 5,00 % |
| | Ginko Biloba Extract | | 0,70 % |
| | Polyglycose | | 0,20 % |
| | Disodium EDTA | | 0,20 % |
| | Citric Acid | | 0,10 % |
| | Glycerin | | 5,00 % |
| | Ginko Biloba Extract | | 0,70 % |
| C | Tocopheryl Acetate | | 1,00 % |
| | Erfindungsgemäßer Blend 1-15 | Clariant | 1,80 % |
| | Sodium Chloride | | 1,00 % |
| | Aluminium Hydroxide | | 0,30 % |

### Herstellung:

- I: Schmelze A bei etwa 80 °C
- II: Erwärme B auf etwa 80 °C
- III: Bei einer Rührgeschwindigkeit von etwa 300 Umdrehungen/Minute gebe II zu I. Erhöhe die Rührgeschwindigkeit nach und nach auf 500 Umdrehungen/Minute und behalte bis zum Ende der Formulierungsarbeit diese Geschwindigkeit bei. Lasse die Mischung auf 35 °C abkühlen
- IV: Bei 35 °C gebe C unter Rühren zu III und lasse auf Raumtemperatur abkühlen

Die erfindungsgemäßen Zusammensetzungen der Beispiele 1 - 15 tragen in den kosmetischen Formulierungen A - M zur Erhöhung der Biostabilität bei.

## Patentansprüche

1. Flüssige Zusammensetzung enthaltend
a) von 40 bis 99,9 Gew.-%, bevorzugt von 45 bis 99,5 Gew.-%, besonders bevorzugt von 50 bis 99 Gew.-% und insbesondere bevorzugt von 55 bis 98 Gew.-% Sorbitanmonocaprylat und
b) von 0,1 bis 60 Gew.-%, bevorzugt von 0,5 bis 55 Gew.-%, besonders bevorzugt von 1 bis 50 Gew.-% und insbesondere bevorzugt von 2 bis 45 Gew.-% an einem oder mehreren antimikrobiellen Wirkstoffen ausgewählt aus der Gruppe bestehend aus den Komponenten b3):
Isothiazolinone ausgewählt aus wässrigen Zusammensetzungen, enthaltend von 20 bis 80 Gew.-% an Methylisothiazolinon, Chloromethylisothiazolinon, einer Mischung aus Methylisothiazolinon und Chloromethylisothiazolinon im Verhältnis 1:3 und/oder Benzylisothiazolinon.

2. Flüssige Zusammensetzung gemäss Anspruch 1 , **dadurch gekennzeichnet, dass** der eine oder die mehreren antimikrobiellen Wirkstoffe der Komponente b3) ausgewählt sind aus der Gruppe Isothiazolinone ausgewählt aus wässrigen Zusammensetzungen enthaltend von 30 bis 70 Gew.-% und bevorzugt von 40 bis 60 Gew.-% an Methylisothiazolinon, Chloromethylisothiazolinon, einer Mischung aus Methylisothiazolinon und Chloromethylisothiazolinon im Verhältnis 1 :3 und/oder Benzylisothiazolinon.

3. Flüssige Zusammensetzung gemäss Anspruch 1 oder 2, **dadurch gekennzeichnet, dass** der eine oder die mehreren antimikrobiellen Wirkstoffe der Komponente b3) ausgewählt sind aus der Gruppe der wässrigen Zusammensetzungen, enthaltend von 30 bis 70 Gew.-% und bevorzugt von 40 bis 60 Gew.-% Methylisothiazolinon.

4. Flüssige Zusammensetzung gemäss einem oder mehreren der Ansprüche 1 bis 3, **dadurch gekennzeichnet, dass** sie ein oder mehrere weitere Substanzen ausgewählt aus d) Wasser, e) antimikrobiellen Wirkstoffen und f) Hydrotropen enthält, wobei die antimikrobiellen Wirkstoffe der Komponente e) und die Hydrotrope der Komponente f) zu den antimikrobiellen Wirkstoffen der Komponente b) unterschiedlich sind.

5. Flüssige Zusammensetzung gemäss einem oder mehreren der Ansprüche 1 bis 4, **dadurch gekennzeichnet, dass** sie einen oder mehrere weitere antimikrobielle Wirkstoffe, die zu den antimikrobiellen Wirkstoffen der Komponente b) unterschiedlich sind, in einer Menge von 0,5 bis 50 Gew.-%, bevorzugt von 5 bis 45 Gew.-% und besonders bevorzugt von 10 bis 45 Gew.-% enthält.

6. Flüssige Zusammensetzung gemäss einem oder mehreren der Ansprüche 1 bis 5, **dadurch gekennzeichnet, dass** sie ein oder mehrere weitere Additive enthält.

7. Flüssige Zusammensetzung gemäss einem oder mehreren der Ansprüche 1 bis 6, **dadurch gekennzeichnet, dass** sie ein klares Aussehen besitzt.

8. Verwendung einer flüssigen Zusammensetzung nach einem oder mehreren der Ansprüche 1 bis 7 zum Konservieren von kosmetischen, dermatologischen oder pharmazeutischen Produkten, vorzugsweise von Cremes, Cremegelen, Lotionen, Shampoos, Duschbädern, Deodorantien, Antiperspirantien, Feuchttüchern (wet wipes), Sonnenschutzformulierungen oder dekorativen Kosmetikartikeln.

9. Verwendung einer flüssigen Zusammensetzung nach einem oder mehreren der Ansprüche 1 bis 7 zur Herstellung von kosmetischen, dermatologischen oder pharmazeutischen Produkten und vorzugsweise von kosmetischen, dermatologischen oder pharmazeutischen Formulierungen.
